# EUROPÄISCHE PATENTSCHRIFT

(11) **EP 0 383 857 B1**
(45) Veröffentlichungstag und Bekanntmachung des Hinweises auf die Patenterteilung: **13.10.1993**
(21) Anmeldenummer: 89907069.2
(22) Anmeldetag: 23.06.1989
(51) Int. Cl.: G01N 1/28, G01N 33/48, B01L 3/14

(54) **VORRICHTUNG ZUM ABSCHEIDEN DES DURCH ZENTRIFUGATION EINES PROBENRÖHRCHENS VON DEM BLUTKUCHEN SEPARIERTEN SERUMS**
DEVICE FOR EXTRACTING THE SERUM SEPARATED BY CENTRIFUGATION IN A SAMPLE TUBE FROM BLOOD
DISPOSITIF D'EXTRACTION DU SERUM SEPARE DE LA MASSE DU SANG PAR CENTRIFUGATION D'UNE EPROUVETTE

(30) Priorität: 24.06.1988 DE 8808138 U
(43) Veröffentlichungstag der Anmeldung: 29.08.1990
(73) Patentinhaber: Ballies, Uwe Werner, Dr., 24103 Kiel (DE)
(72) Erfinder: Ballies, Uwe Werner, Dr., 24103 Kiel (DE)
(74) Vertreter: Tönnies, Jan G., Dipl.-Ing.
(86) Internationale Anmeldenummer: DE8900413
(87) Internationale Veröffentlichungsnummer: WO8912812

(56) Entgegenhaltungen:
- FR-A- 2 383 710
- US-A- 3 865 731

## Beschreibung

Die Erfindung betrifft eine Vorrichtung zum Abscheiden von durch Zentrifugation separiertem Serums mit den Merkmalen des ersten Teiles des Anspruchs 1.

Bei einer solchen Vorrichtung wird zum Einbringen des Serums in einen besonderen Serumbehälter ein Gummistopfen von einer Kanüle durchstoßen, die auf einen auf den Gummistopfen aufzuschiebenden Aufsatz aufgesetzt ist. In die den Gummistopfen durchdringende Kanüle wird ein an einen Serumbehälter angesetzes Saugrohr eingeführt. Sodann wird mittels einer besonderen Vakuumpumpe Serum aus dem Probenröhrchen in den Serumbehälter gepumpt. Diese Vorrichtung hat den Nachteil, daß das Serum mittels einer besonderen Pumpe nach dem Zentrifugieren aus dem Probenröhrchen in den Serumbehälter eingepumpt werden muß.

Aus der FR-A-23 83 710 ist ein Probenröhrchen zur Aufnahme einer durch Zentrifugation im Blutkuchen und Serum zu trennenden Blutprobe bekannt, das einen Trennkörper aufnimmt, dessen Spezifisches Gewicht zwischen denen der zu trennenden Komponenten des Blutes liegt und der sich bei Zentrifugieren zwischen die beiden zu trennenden Komponenten anordnet und nach Beendigung des Zentrifugierens dieser durch Absperrens des Querschnitts des Trennröhrchens zuverlässig voneinander trennt. Diese Ausbildung eines Probenröhrchens ist in der Handhabung besonders einfach. Als wegen des Aerosoleffekts nachteilig kann es dabei jedoch empfunden werden, daß der Probenbehälter bei diesem System bei dem Zentrifugieren nicht geschlossen ist.

Die US-A-3 865 731 zeigt ein Probenröhrchen, in das ein Serumbehälter nach dem Separieren manuell eingedrückt wird, wobei Serum zwischen der Wandung des Röhrchens und der des Serumbehälters aufsteigt und in diesen eindringt.

Der Erfindung liegt die Aufgabe zugrunde, eine Vorrichtung der eingangs genannten Art zu schaffen, bei dem das Serum nach dem Zentrifugieren automatisch in den gesonderten Serumbehälter eingebracht wird.

Erfindungsgemäß wird diese Aufgabe durch die im zweiten Teil des Hauptanspruchs angegebenen Merkmale gelöst. Die Unteransprüche geben bevorzugte Ausgestaltungen der Erfindung an.

Der erfinderische Grundgedanke besteht darin, eine Vorrichtung zu schaffen, bei der - anders als bei dem Stand der Technik - das Serum nicht in einem besonderen Arbeitsvorgang aus dem Probenröhrchen herausgepumpt werden muß, sondern selbsttätig nach Abschluß des Zentrifugierens durch einen bei dem Zentrifugieren entstehenden Überdruck in das besondere Serumgefäß gedrückt wird. Dieses Grundprinzip kann auf vielfältige Weise gelöst werden. Insbesondere kommen auch andere Ausbildungen der Passage in Betracht, durch die das Serum durch die Kanüle in den Serumbehälter gelangt. Dabei ist jedoch zu beachten, daß die Passage derart auszubilden ist, daß eine Komunikation zwischen dem Raum zwischen dem Boden des Serumbehälters und dem Gummistopfen erst bei einer mehr oder weniger vollständigen Absenkung des Serumbehälters während des Zentrifugierens gegeben ist.

Die Erfindung wird im folgenden anhand einer Zeichnung erläutert. Dabei zeigt:
- Fig. 1: eine Schnittdarstellung durch ein Probenröhrchen nach Aufbringen des aber vor dem Zentrifugieren,
- Fig. 2: eine Fig. 1 entsprechende Darstellung nach dem Zentrifugieren.

Auf das Probenröhrchen 10, das durch einen Stopfen 12 verschlossen ist, ist ein Aufsatz 14 aufgesetzt, der aus einem ersten, den Stopfen 12 umfassenden Kragen 16, einer den Stopfen 12 durchdringenden Kanüle 18 und einem zweiten, sich von dem Stopfen 12 weg erstreckenden zweiten Kragen 22 besteht. Der zweite Kragen 22 nimmt einen Serumbehälter 20 kolbenartig in ihm gleitend auf.

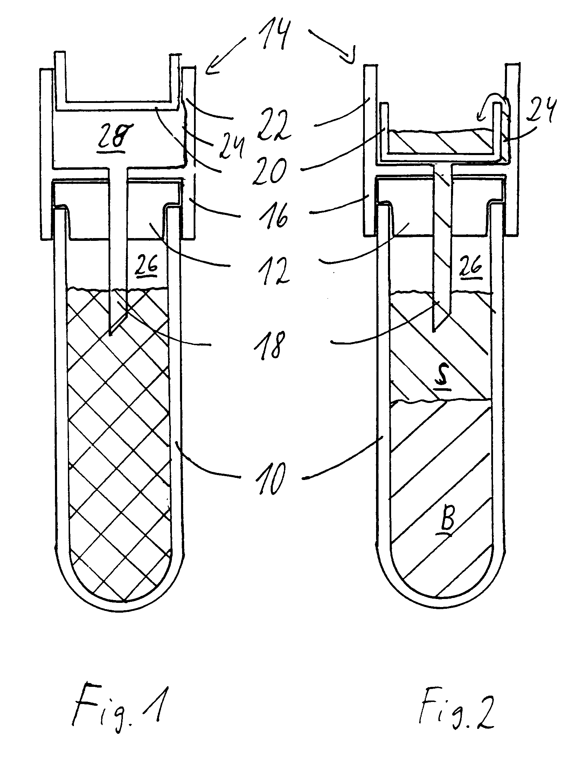

Vor dem Zentrifugieren ist der Serumbehälter 20 mit einem Abstand von dem Gummistopfen 12 angeordnet, so daß sich zwischen Serumbehälter 20 und Gummistopfen 12 ein Raum 28 ausbildet (Fig.1). Bei dem Zentrifugieren senkt sich der Serumbehälter 20 aufgrund der Fliehkräfte in Richtung auf den Gummistopfen 12 ab. Da der Serumbehälter 20 an der Innenwandung des zweiten Kragens 22 dichtend anliegt, wird die Luft in dem Raum 28 durch den Serumbehälter 20 verdrängt. Die Luft in dem Raum 28 wird dabei durch die Kanüle 18, die den Gummistopfen 12 durchstößt, in das Probenröhrchen 10 eingepreßt, wodurch in dem Probenröhrchen ein Überdruck entsteht.

Dieser Überdruck in dem Probenröhrchen 10 bewirkt, daß in der Kanüle 18 das sich oben absetzende Serum S über den Pegel des Serums außerhalb der Kanüle 18 steigt, das weitere Steigen jedoch durch die Fliehkraft verhindert wird. Während des Zentrifugierens wirkt die Fliehkraft, die die Flüssigkeitssäule in der Kanüle 18 nach außen, also zu dem geschlossenen Ende des Probenröhrchens 10 hin drückt, gegen den Druck, der von der Luft in den Raum 26 in dem Probenröhrchen auf das Serum ausgeübt wird. Nach Beendigung des Zentrifugierens dagegen bewirkt die sich in dem die Kanüle 18 umgebenden Raum 26 des Probenröhrchens unter Druck stehende Luft ein Fördern des Serums durch die sich bis in den Bereich des abgesetzten Serums erstreckende Kanüle 18 durch diese nach oben durch den Gummistopfen 12 hindurch. Eine bei dem gezeigten Ausführungsbeispiel als Kerbe in der Wandung des zweiten Kragens 22 des Aufsatzes 14 sich achsenparallel gerade bis über die Oberkante des Serumbehälters 20 erstreckende Passage 24, die erst bei abgesenktem Serumbehälter 20 eine Verbindung zwischen der Kanüle 18 und dem Serumbehälter 20 freigibt, erlaubt ein Eintreten des aus dem Probenröhrchen 10 herausgedrückten Serums in den von dem zweiten Kragen 14 aufgenommenen Serumbehälter 20. Das Serum wird jetzt von dem gesonderten Serumbehälter 20 aufgenommen, das Probenröhrchen 10 kann in ein Analysengerät eingesetzt werden, ohne daß zuvor ein besonderer Arbeitsschritt des Einbringens des Serums in ein gesondertes Serumgefäß erforderlich wäre.

Es versteht sich, daß die Dicke der Kanüle derart zu wählen ist, daß der erfindungsgemäß genutzte Überdruckeffekt bewirkt wird.

Die Kanüle kann auch - anders als bei dem dargestellten Ausführungsbeispiel - an den Boden des Serumbehälters 20 angesetzt sein, wenn die Kanüle 18 im Bereich des Bodens des Serumbehälters 20 mit einer Ausnehmung versehen ist, die bei dem Absenken des Serumbehälters ein Verdrängen der Luft in dem Raum 28 durch die Kanüle hindurch in das Probengefäß und ein Aufsteigen des Serums durch die Kanüle 18 unterhalb des Bodens des Serumbehälters 20 durch die Passage in den Serumbehälter 20 hinein erlaubt.

Die weiter vorgeschlagene Ausbildung der Passage 24 derart, daß diese Fibrinfasern oder dgl. zurückhaltend ausgebildet ist, kann durch eine geeignete Rauhigkeit oder Struktur des Bodens des Serumbehälters 20 bzw. der oberen Fläche des auf den Stopfen aufgesetzten Wandung bewirkt werden.

## Patentansprüche

1. Vorrichtung zum Abscheiden des durch Zentrifugation eines wenigstens einseitig durch einen Stopfen aus Gummi oder dgl. verschlossenen Probenröhrchens von dem Blutkuchen separierten Blutserums, bestehend aus einem auf den Stopfen (12) aufgesetzten, im wesentlichen rotationsymmetrischen Aufsatz (14), der mit einem den Stopfen (12) umfassenden ersten Kragen (16) und einer den Stopfen (12) durchdringenden Kanüle (18) versehen ist, und mit einem zur Aufnahme eines Teiles des sich in dem Probenröhrchen (10) bei der Zentrifugation absetzenden Serums (S) dienenden Serumbehälter (20),
**dadurch gekennzeichnet, daß**
der Aufsatz (14) mit einem zweiten, sich von dem Stopfen (12) weg erstreckenden Kragen (22) versehen ist, der den Serumbehälter (20) kolbenartig in ihm gleitend aufnimmt,
die Kanüle (18) sich bis in das Serum (S) erstreckend ausgebildet ist,
der zweite Kragen (22) oder der Serumbehälter (20) mit einer von der Kanüle zu dem Serumbehälter (20) führenden Passage (24) versehen ist, die erst bei an den Stopfen (12) abgesenktem Serumbehälter (20) freigeben ist,
wobei
der Serumbehälter (20) vor dem Zentrifugieren mit einem Abstand vom Stopfen (12) angeordnet ist, so daß sich zwischen Serumbehälter (20) und Stopfen (12) ein Raum (28) ausbildet,
der Serumbehälter (20) bei dem Zentrifugieren aufgrund der Fliehkräfte in Richtung auf den Stopfen (12) gleiten kann, und
nach Beendigung des Zentrifugierens über die Passage (24) ein Eintreten des aus dem Probenröhrchen herausgedrückten Serums (S) in den Serumbehälter (20) erfolgen kann.

2. Vorrichtung nach Anspruch 1, dadurch gekennzeichnet, daß die Passage (24) als eine sich in der Innenwandung des zweiten Kragens (22) bei an den Stopfen (12) abgesenktem Serumbehälter (20) sich bis über dessen Oberkante achsenparallel erstreckende Kerbe ausgebildet ist.

3. Vorrichtung nach Anspruch 1 oder Anspruch 2, dadurch gekennzeichnet, daß die Passage (24) mit Filtereinrichtungen versehen ist, die Fibrinfasern und dgl. zurückhalten.

4. Vorrichtung nach einem der vorangehenden Ansprüche, gekennzeichnet durch eine den Serumbehälter (20) verschließende Kappe.

## Claims

1. Apparatus for removing the blood serum separated from the blood clot by centrifuging a test tube sealed on at least one side by a stopper made from rubber or the like and comprising a substantially rotationally symmetrical future (14) feed to the stopper (12) and which is provided with a first collar (16) surrounding the stopper (12) and a cannula (18) penetrating the stopper (12) and with a serum container (20) used for receiving pert of the serum (S) deposited in the test tube (10) during centrifuging, characterized in that the future (14) is provided with a second collar (22) extending away from the stopper (12) and which receives the serum container (20) which slides in piston-like manner therein, the cannula (18) extends into the serum (S), the second collar (22) or the serum container (20) is provided with a passage (24) leading from the cannula to the serum container (20) and which is only freed with the serum container (20) lowered at the stopper (12) and in which the serum container (20) is spaced from the stopper (12) prior to centrifuging, so that a gap (28) is formed between the serum container (20) and the stopper (12), as a result of the centrifugal forces during centrifuging the serum container (20) can slide towards the stopper (12) and at the end of centrifuging by means of the massage (24) the serum (S) forced out of the test tube can enter the serum container (20).

2. Apparatus according to claim 1, characterized in that the passage (24) is constructed as a notch in the inner wall of the second collar (22) and which when the serum container (20) is lowered to the stopper (12) extends axially parallel over the upper edge thereof.

3. Apparatus according to claim 1 or 2, characterized in that the passage (24) is provided with filter means, which retain fibrin fibres and the like.

4. Apparatus according to one of the preceding claims, characterized by a cap sealing the serum container (20).

## Revendications

1. Dispositif de séparation du sérum sanguin débarrassé du gâteau sanguin, par centrifugation d'un tube éprouvette obturé au moins à une extrémité par un bouchon en caoutchouc ou analogue, composé d'un socle (14), sensiblement symétrique de rotation, posé sur les bouchons (12), et pourvu d'une première collerette (16), entourent le bouchon (12) et d'une canule (18), traversant le bouchon (12), et avec un récipient à sérum (20) servant à recevoir une partie du sérum (S) se déposant dans les tubes éprouvettes (10) lors de la centrifugation, caractérisé en ce que :
le socle (14) est pourvu d'une deuxième collerette (22), allant en s'écartant du bouchon (12), y recevant, coulissant à la façon d'un piston, le récipient à sérum (20),
la canule (18) est réalisée en s'étendent jusque dans le sérum (S),
la deuxième collerette (22) ou le récipient à sérum (20) est pourvu(e) d'un passage (24) menant de la canule au récipient à sérum (20), passage qui n'est dégagé que lorsque le récipient à sérum (20) est abaissé sur le bouchon (12),
le récipient à sérum (20) étant disposé, avant la centrifugation, à une certaine distance du bouchon (12), de manière qu'un espace soit créé entre le récipient à sérum (20) et le bouchon (12),
le récipient à sérum (20) pouvant, lors de la centrifugetion, coulisser du fait des efforts centrifuges, en direction du bouchon (12),
une pénétration, par le passage (24), dans le récipient A sérum (20), du sérum (S) expulsé des tubes échantillons pouvant s'effectuer, après achèvement de la centrifugation.

2. Dispositif selon la revendication 1, caractérisé en ce que le passage (24) est réalisé sous forme d'une entaille, ménagée dans la paroi intérieure de la deuxième collerette (22) et s'étendent parallèlement à l'axe, jusqu'à atteindre l'arête supérieure du récipient à sérum, lorsque le récipient à sérum (20) est abaissé sur le bouchon (12).

3. Dispositif selon la revendications 1 ou 2, caractérisé en ce que le passage (24) est pourvu de dispositifs filtrants, retenant les fibres de fibrine et analogue,

4. Dispositif selon l'une des revendications précédentes, caractérisé par un capuchon obturant le récipient à sérum (20).
